# NEUE EUROPÄISCHE PATENTSCHRIFT

(11) **EP 0 523 506 B2**
(45) Veröffentlichungstag und Bekanntmachung des Hinweises auf die Entscheidung über den Einspruch: **15.01.2003**
(45) Hinweis auf die Patenterteilung: 01.10.1997
(21) Anmeldenummer: 92111445.0
(22) Anmeldetag: 06.07.1992
(51) Int. Cl.: A61C 1/00, G02B 6/36

(54) **Handstück zur stomatologischen Applikation von Laserlicht**
Handpiece for stomatological use of laser light
Pièce à main pour application stomatologique de lumière de laser

(30) Priorität: 17.07.1991 DE 4123729
(43) Veröffentlichungstag der Anmeldung: 20.01.1993
(73) Patentinhaber: SIEMENS AKTIENGESELLSCHAFT, 80333 München (DE)
(72) Erfinder: Franetzki, Manfred, Dr., W-6140 Bensheim (DE)
(74) Vertreter: Sommer, Peter

(56) Entgegenhaltungen:
- EP-A- 0 073 617
- EP-A- 0 369 043
- EP-A- 0 392 951
- WO-A-91/16006
- FR-A- 2 598 313
- JP-A- 6 007 689
- US-A- 3 821 510
- US-A- 3 865 113
- US-A- 4 541 802
- US-A- 4 608 980
- US-A- 4 625 724
- US-A- 4 826 431

## Beschreibung

Die Erfindung bezieht sich auf ein Handstück zur stomatologischen Applikation von Laserlicht, insbesondere zum Abtragen von Zahnhartsubstanz, enthaltend ein Basisteil mit einer Griffhülse, welches auf der proximalen Seite mit einem Versorgungsschlauch zur Zufuhr von Licht und Kühlmedium verbindbar ist und welches auf der distalen Seite ein Kopfgehäuse enthält, an dem mittels eines Trägers ein Applikationselement gehaltert ist, welches eine mit einer Lichtaustrittsstelle versehene Arbeitsspitze aufweist, und enthaltend ferner Mittel zur Übertragung des Laserlichts vom Basisteil zur Lichtaustrittsstelle.

In verschiedenen Patentdokumenten (US-3 865 113, US-3 821 510, EP-A-0 673 617, DE-OS 3 713 512, WO-US 89/00984, WO-US 89/03825 und EP-A3-0 392 951) werden Lasersysteme beschrieben, die sich unter anderem zur Zahnbehandlung eignen sollen. Bei einigen der dort offenbarten Ausführungsformen tritt das Laserlicht, gegebenenfalls nach in der Regel einmaliger Umlenkung im Handstückkopf, direkt als Freistrahl aus, bei anderen wird das Laserlicht in einen am Handstückkopf angeordneten, optisch leitenden Applikator geleitet, an dessen Spitze es dann gerade, d.h. in Richtung der Längsachse des Applikators, austritt. Diese Art der Handhabung entspräche einem Bohren mit der Stirnfläche eines zylindrischen Bohrers nach herkömmlicher Weise. Nachdem bekanntlich aber mit der Mantelfläche eines Werkzeuges präpariert wird, würde die Handhabung nach den bekannten Laserhandstücken ein völliges Umlernen des Zahnarztes notwendig machen. Außerdem würden bestimmte Geometrien sich nicht oder nur sehr mühsam mit dieser Handhabung erzeugen lassen, z.B. Seitenwände oder besondere Hinterschnitte in Kavitäten. Des weiteren würden Präparationen im Interdentalraum oder von lingualen Kavitäten schwer durchführbar sein. Ein weiterer Nachteil der bekannten Systeme ist, daß mit einem Präparieren im Freistrahl keine glatten Oberflächen und scharfe Konturen zu erzielen sind und überdies das Risiko einer ungewollten Verletzung besteht.

Aus der FR 2 598 313 ist ein zahnärztliches Handinstrument bekannt, bei welchem eine schwenkbare Arbeitsspitze für den Austritt von Laserlicht vorgesehen ist. Das Laserlicht tritt hier nach einer Umlenkung aus dem Handstück in die Arbeitsspitze ein, wobei hierzu ein Reflektor verwendet wird. In welcher Art und Weise das Licht aus der Arbeitsspitze austritt, ist nicht bekannt. Aus der JP 2-71516 ist ein zahnärztliches Handinstrument bekannt, welches ein Applikationselement mit einer im wesentlichen in Längsrichtung der Längsachse des Applikationselements über einen großen Winkel verstreut austritt. Der Lichtaustrittsfächer erstreckt sich dabei schräg zur Längsachse des Applikationselements. Aus der JP 62-202 815 ist ein weiteres zahnärztliches Handstück bekannt, dessen Applikationselement für Laserstrahlen etwa um 60° gegenüber der Längsachse des Handstücks geneigt angeordnet ist. Aus der Spitze des Applikationselements tritt quer zur Längsachse des Applikationselements, also seitlich, Licht aus.

Der im Anspruch 1 angegebenen Erfindung liegt die Aufgabe zugrunde, demgegenüber eine Verbesserung zu erzielen, insbe sondere ein Handstück anzugeben, mit dem der Benutzer Präparationen im Patientenmund in der ihm bisher gewohnten Weise durchführen kann, wobei mit dem Handstück das Laserlicht konzentriert auf die zu bearbeitende Präparationsstelle gebracht werden kann. Des weiteren ist Sorge dafür zu treffen, daß die Präparationsstelle mit einem geeigneten Kühlmedium (Wasser oder Spray) versorgt und außerdem das Arbeitsfeld entsprechend ausgeleuchtet werden kann.

Ein wesentlicher Vorteil der nachfolgend anhand mehrerer Ausführungsformen beschriebenen Erfindung ist, daß mit dem Handstück ein ähnliches Arbeiten wie mit konventionellen Instrumenten, insbesondere mit den bekannten Hand- und Winkelstückbohrern, erzielbar ist.

Es zeigen:
Figur 1 den vorderen Teil des erfindungsgemäßen Handstückes ohne aufgesetztem Applikationselement,
Figur 2 den hinteren Teil des Handstückes, verbunden mit einem Anschlußteil,
Figur 3 eine Variante zur dem in Figur 1 gezeigten Träger für ein Applikationselement,
Figur 4 den oberen Teil einer ersten Ausführungsform eines Applikationselements,
Figur 5 den unteren Teil des Applikationselements nach Figur 4 in verkleinerter und vereinfachter Darstellung in verschiedenen Ansichten,
Figur 6 eine weitere Ausführungsform eines Applikationselements in vereinfachter Darstellung in verschiedenen Ansichten.

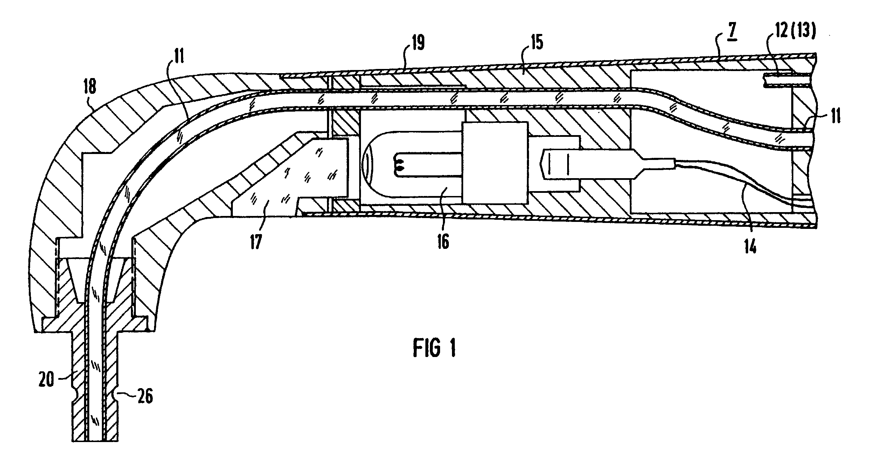

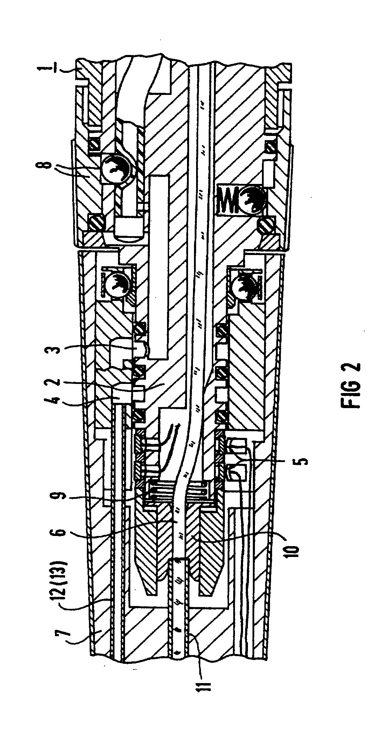

Anhand der Figuren 1 und 2 wird der Aufbau des erfindungsgemäßen Handstückes näher erläutert, wobei mit der Beschreibung der Figur 2 begonnen wird, die den rückwärtigen Teil des Handstückes mit einem daran angeschlossenen Anschlußteil zeigt.

Mittels eines nicht näher dargestellten Versorgungsschlauches werden die Medien Luft, Wasser und elektrischer Strom sowie Licht mittels dafür geeigneter Leitungen an eine allgemein mit 1 bezeichnete Anschlußarmatur herangeführt. Die Anschlußarmatur 1 ist in bekannter Weise als Drehkupplung ausgebildet und enthält ein zapfenförmiges Kupplungsteil 2, an dem in bekannter Weise über Leitungen und Kanäle 3 bis 6 die Medien Wasser, Luft, elektrischer Strom und Licht von der Anschlußarmatur 1 auf das Handstück 7 übertragen werden. Mit 8 ist eine in die Wasserzuleitung eingeschaltete Dosiereinrichtung bezeichnet, mit der durch Quetschen des Zuleitungsschlauches der Durchsatz des Wassers geregelt werden kann. Die Führung der zur Kühlung der Präparationsstelle dienenden Medien Luft und Wasser erfolgt in der bei Bohrhandstücken üblichen Weise, so daß im Rahmen dieser Beschreibung nicht weiter darauf eingegangen zu werden braucht.

Der Lichtleiter 6 dient zur Übertragung von Laserlicht, das von einem extern angeordneten Laserlichtgenerator erzeugt wird. Der Lichtleiter 6 endet stirnseitig des Kupplungsteils 2 in einem axial beweglichen und durch eine Druckfeder 9 belasteten Anschlußteil 10. Die Weiterführung des Laserlichts im Handstück erfolgt mittels eines in einem Hüllrohr angeordneten Lichtleiters, der beim Aufsetzen des Handstückes 7 auf die Anschlußarmatur 1 mit dem stirnseitigen Ende des Lichtleiters 6 so in Eingriff kommt, daß eine Lichtübertragung gewährleistet ist. Zum leichteren Ankoppeln ist das Anschlußteil 10 stirnseitig facettiert. Um optische Verluste an der Lichtkupplungsstelle zu vermeiden bzw. möglichst klein zu halten, ist es zweckmäßig, vor dem Ankuppeln zumindest die eine Stirnseite des Lichtleiterendes mit einem gut lichtleitenden Medium, z.B. mit einer geeigneten Flüssigkeit, zu versehen.

Der Lichtleiter 11 sowie weitere Leitungen 12 bis 14 für die Medien Luft/Wasser und Strom sind in einem Basisteil 15 entsprechend geführt bzw. gehaltert. Im Basisteil 15 ist auch eine zur Ausleuchtung der Präparationsstelle dienende Glühlampe 16 gehaltert, die über die Leitungen 14 mit den Schleifringen 5 in der Anschlußarmatur 1 verbunden ist. Die Gühlampe 16 korrespondiert mit einer das Licht auf die Präparationsstelle lenkenden Optik 17. Das etwa rechtwinkelig zur Handstücklängsachse abgewinkelte Kopfgehäuse 18 ist mit dem Basisteil 15 des Handstückes in geeigneter Weise verbunden. Mit Vorteil kann diese Verbindung auch als leicht lösbare Rastverbindung ausgebildet sein; dementsprechend wäre dann an dieser Trennstelle auch eine geeignete Trennkupplung für den Lichtleiter vorzusehen. Im Rahmen dieser Maßnahmen ist es vorteilhaft, die das Basisteil 15 umgebende Griffhülse 19 abnehmbar anzuordnen. Die Griffhülse könnte so z.B. zusammen mit dem Kopfgehäuse 18 zu Sterilisationszwecken vom Basisteil 13 leicht getrennt werden.

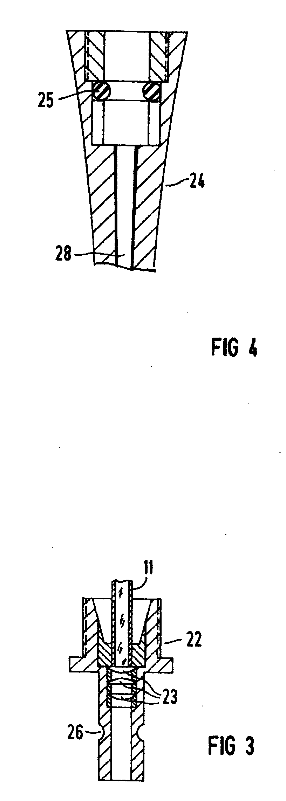

Das freie Ende des Lichtleiters 11 endet in einem Träger 20, auf den die in den Figuren 4 bis 11 gezeigten Applikationselemente alternativ aufsetzbar sind. Der Träger 20 ist stirnseitig des Kopfgehäuses 18 eingeschraubt, kann also bei Bedarf leicht ausgewechselt werden. Demgemäß ist das Ende des Lichtleiters 11 im Träger 20 mit Spiel geführt.

Der innerhalb des Handstückes verwendete Lichtleiter kann wahlweise und je nach der Wellenlänge des vorgesehenen Lasertyps ein flexibles oder starres Hohlrohr sein, das innen poliert oder beschichet ist. Ebenso kann es auch eine starre oder flexible Lichtfaserleitung mit einem für die betreffende Wellenlänge durchlässigen Material sein. Für Wellenlängen von 250 nm bis ca. 2,7 µm kann vorteilhafterweise Quarzglas, für Wellenlängen über ca. 2,5 ZrF oder ein Silberhalogenid zur Anwendung kommen. Ebenso können flüssigkeitsgefüllte Hohlleiter in Betracht gezogen werden.

Der Lichtleiter 11 kann - wie in Figur 1 dargestellt - bis zum freien Ende des Trägers 20 geführt sein; alternativ kann auch, wie in Figur 3 dargestellt, ein Träger 22 vorgesehen sein, in dem der Lichtleiter 11 endet und an dessen Ende sich eine das Licht bündelnde Optik 23 anschließt.

Auf den Träger 20 bzw. 22 können nun alternativ diverse Applikationselemente aufgesetzt werden, wie sie anhand der nachfolgenden Figuren noch näher erläutert werden. Die Figur 4 zeigt ein erstes Applikationselement 24, welches mittels einer O-Ring-Verbindung 25, 26 am Träger 21 bzw. 22 drehund leicht abnehmbar gerastet werden kann. Anstelle der O-Ring-Verbindung, die einen gewissen, unter Umständen gewünschten Reibschluß aufweist, kann auch ein Federring oder ein anders geeigneter Rastelement vorgesehen werden.

Das Applikationselement 24 weist einen sich zur Arbeitsspitze 27 hin konisch verjüngenden, innen verspiegelten Hohlkanal 28 auf, der das Licht von Lichtleiter 11 übernimmt und an einem Lichtaustrittsfenster 29 austreten läßt. Anstelle des Hohlkanals kann alternativ auch ein angeordneter Glasstab, der an der Arbeitsspitze ein zentrisches Lichtaustrittsfenster bildet, vorgesehen werden.

Sowohl bei der in Figur 4 dargestellten Ausführungsform als auch in den folgenden Abbildungen aufgezeigten Ausführungsformen ist die Arbeitsspitze des Applikationselements so gestaltet, daß sich der Fokus der Lichtstrahlung außerhalb, jedoch unmittelbar vor dem Lichtaustrittsfenster des Applikationselements bildet oder die Anordnung ist so getroffen, daß das Laserlicht divergierend aus dem Lichtaustrittsfenster austritt. In diesem Falle ist die größte Energiedichte direkt in der Fensterebene. Eine Fokussierung unmittelbar vor dem Lichtaustrittsfenster kann z.B. durch eine spiegelnde Rückwand oder durch eine Linse im Austrittsfenster erreicht werden, wie dies aus den nachfolgenden Abbildungen hervorgeht.

Durch die vorgeschlagenen Maßnahmen kann erreicht werden, daß wie mit einem handelsüblichen Bohrinstrument berührend, d.h. mit taktiler Rückmeldung, gearbeitet werden kann, und zwar nur in unmittelbarer Nähe der Spitze. Außerhalb der Spitze fällt die divergierende Lichtenergie wegen des Strahlenganges sehr schnell ab, so daß eine Beschädigung des Umfeldes der Präparationsstelle verhindert werden kann.

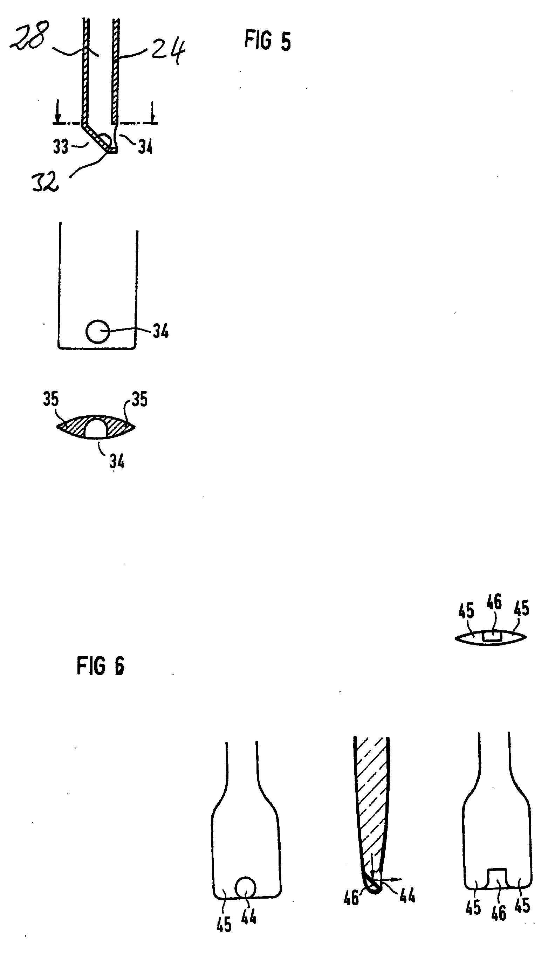

Die Figur 5 zeigt eine Ausführungsform einer Arbeitsspitze, also vom unteren Teil eines Applikationselements, und zwar im Längsschnitt, darunter in einer Ansicht auf die Lichtaustrittsstelle und schließlich im Querschnitt entlang der durch die Pfeile angegebenen Linie im Bereich der Lichtaustrittsstelle. Bei der Ausführungsform handelt es sich um einen Hohlleiter, der innen beschichtet (verspiegelt) oder poliert ist. Die in Figur 5 dargestellte Arbeitsspitze 33 weist eine quer zur Längsachse angeordnete Lichtaustrittsstelle 34 auf, die für ein mantelseitiges Bearbeiten der Präparationsstelle geeignet ist. Das Licht wird hier an einer schrägen Fläche 32 umgelenkt. Die Fläche 32 kann vorteilhafterweise derartige optische Eigenschaften aufweisen, daß sie das Licht an der Umlenkstelle auf den an bzw. unmittelbar vor der Austrittsstelle befindlichen Fokus bündelt.

Beidseitig des Lichtaustrittsfensters 34 sind flügelartige Führungsfähnchen 35 angeordnet, die es erlauben, wenn die Spitze einmal auf die Präparationsfläche aufgesetzt ist, daß sich dann die Lichtaustrittsöffnung beim Weitergleiten auf der Bearbeitungsoberfläche stets in Richtung auf diese Oberfläche ausrichtet. Auf diese Weise kann z.B. eine Zahnkavität oder ein Kronenstumpf ohne Nachstellen der Öffnung von Hand kontinuierlich bearbeitet werden.

Die Figur 6 zeigt eine Ausführungsform einer Arbeitsspitze, die ähnlich aufgebaut ist wie die zuvor beschriebene, bei der jedoch das gesamte Applikationselement aus einem das Laserlicht durchlassenden Vollmaterial besteht und die Oberflächen bis auf die Lichtaustrittsstelle verspiegelt sind. Die Arbeitsspitze ist also so beschaffen, daß das Licht nur an der gewünschten Öffnung, also am Austrittsfenster, austreten kann. Erreicht wird dies dadurch, daß an den anderen Flächen das Licht durch Totalreflexion oder - wie gezeigt - durch Reflexion an einer verspiegelten Oberfläche in das Material der Spitze zurückgeworfen wird. In der Figur ist das (jeweils ebene) Lichtaustrittsfenster mit 44 bezeichnet.

Die Ausführungsform nach Figur 6 enthält, wie bei Figur 5, beidseitig der Austrittsfenster angeordnete, flügelartige Führungselemente 45. Mit 46 ist die die Strahlen umlenkende schräge Fläche bezeichnet, die außen ebenfalls verspiegelt ist.

Wenngleich in den vorliegenden Ausführungsbeispielen der Lichtaustritt exakt axial oder radial beschrieben ist, so sind im Rahmen der Erfindung Ausführungen möglich, bei denen der Lichtstrahl zwischen den beiden vorgenannten Positionen austreten kann.

Die Lichtaustrittsöffnung kann bei allen Versionen punktoder schlitzförmig sein, wobei letztere Gestaltung besonders vorteilhaft ist für das Bearbeiten eines Kronenstumpfes.

Abschließend sei darauf hingewiesen, daß die Präparationsstelle in der bei konventionellen Bohrinstrumenten üblichen Weise gekühlt werden kann; die hierzu notwendigen Medien Luft und/oder Wasser wären dann in geeigneter Form an die Arbeitsspitze der Applikationselemente heranzuführen.

## Patentansprüche

1. Handstück zur stomatologischen Applikation von Laserlicht, insbesondere zum Abtragen von Zahnhartsubstanz, enthaltend ein Basisteil (15) mit einer Griffhülse (19), welches auf der proximalen Seite mit einem Versorgungsschlauch zur Zufuhr von Licht und Kühlmedien verbindbar ist und welches auf der distalen Seite ein Kopfgehäuse (18) enthält, an dem mittels eines Trägers (20, 22) ein Applikationselement (24) gehaltert ist, welches eine mit einer Lichtaustrittsstelle (31, 34, 38, 41) versehene Arbeitsspitze (30, 33, 36) aufweist, und enthaltend ferner Mittel (11) zur Übertragung des Laserlichts vom Basisteil zur Lichtaustrittsstelle,
**dadurch gekennzeichnet, dass** das Kopfgehäuse senkrecht zur Handstücklängsachse abgewinkelt ist, dass die Lichtaustrittsstelle (31, 34, 38, 41) im wesentlichen quer zur Längsachse des Applikationselements (24) angeordnet ist, dass das Applikationselement (24) im Bereich des Lichtaustritts optische Mittel (32, 39, 40, 41) enthält, die den Lichtstrahl vor seinem Austritt auf die Lichtaustrittsstelle (31, 34, 38, 41) umlenken und dass die Arbeitsspitze des Applikationselements im Bereich der Lichtaustrittsstelle seitliche Führungselemente (35) aufweist, welche ein selbsttätiges Ausrichten der Lichtaustrittsstelle auf die zu bearbeitende Oberfläche bewirken, wozu das Applikationselement (24) an einem das Ende des Lichtleiters (11) aufnehmenden Halteteil (20) drehbar gehaltert ist.

2. Handstück nach Anspruch 1, **dadurch gekennzeichnet, daß** die optischen Mittel (32, 39, 40, 41) das Laserlicht im Applikationselement (24) so bündeln, dass der Fokus unmittelbar vor dem Lichtaustrittsfenster (31, 34, 25, 38) der Arbeitsspitze liegt.

3. Handstück nach Anspruch 1 oder 2, **dadurch gekennzeichnet, daß** das Halteteil (20 im Kopfgehäuse (18) abnehmbar gehaltert ist.

4. Handstück nach Anspruch 1 oder 2, **dadurch gekennzeichnet, daß** das Halteteil (20) optische Elemente (23) zur Lichtbündelung auf die Lichtführungselemente (28) im Applikationselement (24) enthält.

5. Handstück nach einem der Ansprüche 1 bis 4, **dadurch gekennzeichnet, daß** das Applikationselement als Hohlleiter ausgebildet ist oder einen solchen enthält.

6. Handstück nach einem der Ansprüche 1 bis 5, **dadurch gekennzeichnet, daß** das Applikationselement mehrere, am Umfang angeordnete Lichtaustrittsfenster (38, 41) aufweist, wodurch der Laserlichtstrahl am gesamten Umfang der Arbeitsspitze austreten kann.

7. Handstück nach einem der Ansprüche 1 bis 6, **dadurch gekennzeichnet, daß** das Applikationselement aus einem Laserlicht durchlässigen Vollmaterial besteht (Figuren 9 bis 11).

8. Handstück nach einem der Ansprüche 1 bis 6, **dadurch gekennzeichnet, daß** die optischen Mittel im Bereich der Arbeitsspitze angeordnete, das Laserlicht reflektierende innere Flächen (39, 40) umfassen, die den Fokus unmittelbar vor das Lichtaustrittsfenster (38, 41) der Arbeitsspitze legen.

9. Handstück nach einem der Ansprüche 1 bis 8, **dadurch gekennzeichnet, daß** es dreh- und abnehmbar auf der Anschlußarmatur eines Versorgungsschlauches aufgesetzt ist.

10. Handstück nach Anspruch 9, **dadurch gekennzeichnet, daß** die Anschlußarmatur (1) als Schnellkupplung ausgebildet ist und die erforderlichen Medienübertragungsmittel für das Laserlicht, Luft und gegebenenfalls Wasser sowie elektrische Energie für eine Arbeitsfeldbeleuchtung enthält.

11. Handstück nach Anspruch 10, **dadurch gekennzeichnet, daß** die Übertragung des Laserlichtes innerhalb des Handstückes durch Freistrahl und Umlenkung an optischen Spiegeln mittels Lichtleiter aus Vollmaterial oder aus Hohlrohrleitungen, mittels Flüssigkeitsleitern oder einer Kombination der vorgenannten Übertragungsmittel erfolgt.

## Claims

1. Handpiece for stomatological application of laser light, particularly for eroding hard dental substance, containing a base portion (15) having a gripping sleeve (19), which base portion can be connected, on the proximal side, to a supply tube for supplying light and coolants and contains, on the distal side, a head housing (18), on which there is held by means of a carrier (20, 22) an application element (24), which has a work tip (30, 33, 36) provided with a light output point (31, 34, 38, 41), further containing means (11) for transmitting the laser light from the base portion to the light outlet point, **characterized in that** the head housing is offset at right angles to the longitudinal axis of the handpiece, **in that** the light outlet point (31, 34, 38, 41) is arranged substantially cross-wise with respect to the longitudinal axis of the application element (24), **in that** the application element (24) contains, in the region of the light outlet, optical means (32, 39, 40, 41) which deflect the light beam before it exits onto the light outlet point (31, 34, 38, 41) and **in that** in the region of the light outlet point the work tip of the application element has lateral guide elements (35) which effect an automatic alignment of the light outlet point onto the surface which is to be treated, for which purpose the application element (24) is held rotably on a holding portion (20) which receives the end of the light guide (11).

2. Handpiece according to claim 1, **characterised in that** the optical means (32, 39, 40, 41) focus the laser light in the application element (24) in such a way that the focus lies directly in front of the light outlet window (31, 34, 38) of the work tip.

3. Handpiece according to claim 1 or 2, **characterized in that** the holding portion (20) is detachably held in the head housing (18).

4. Handpiece according to claim 1 or 2, **characterised in that** the holding portion (20) contains optical elements (23) for focusing light onto the light guide elements (28) in the application element (24).

5. Handpiece according to one of the claims 1 to 4, **characterised in that** the application element is constructed as a waveguide or contains such a waveguide.

6. Handpiece according to one of the claims 1 to 5, **characterised in that** the application element has a plurality of light outlet windows (38, 41) arranged at the circumference, as a result of which the laser light beam can exit at the whole circumference of the work tip.

7. Handpiece according to one of the claims 1 to 6, **characterised in that** the application element consists of a solid material which is permeable to laser light (Figures 9 to 11).

8. Handpiece according to one of the claims 1 to 6, **characterised in that** the optical means comprise inner surfaces (39, 40) which are arranged in the region of the work tip and reflect the laser light and place the focus directly in front of the light outlet window (38, 41) of the work tip.

9. Handpiece according to one of the claims 1 to 8, **characterised in that** said handpiece is rotatably and detachably mounted on the connection armature of a supply tube.

10. Handpiece according to claim 9, **characterised in that** the connection armature (1) is constructed as a rapid action coupling and contains the required media-transmission means for the laser light, air and possibly water, as well as electrical energy for a workplace illumination.

11. Handpiece according to claim 10, **characterised in that** the transmission of the laser light inside the handpiece by free beam and deflection at optical mirrors by means of light guides consisting of solid material or of hollow tubes, takes place by means of liquid guides or a combination of the above-mentioned transmission means.

## Revendications

1. Pièce à main pour l'application en stomatologie de lumière laser, notamment pour enlever de l'émail, comportant une pièce (15) de base qui comporte un tube (19) pour la préhension, qui peut être reliée du côté proximal à un tuyau souple d'alimentation servant à envoyer de la lumière et du fluide de refroidissement et qui comporte du côté distal un boîtier (18) de tête, auquel est fixé, au moyen d'un support (20, 22), un élément (24) d'application, qui comporte une pointe (30, 33, 36) de travail munie d'un point (31, 34, 38, 41) de sortie de la lumière, et comportant de plus des moyens (11) de transmission de la lumière laser de la pièce de base au point de sortie de la lumière, **caractérisée en ce que** le boîtier de tête est coudé perpendiculairement à l'axe longitudinal de la pièce à main, le point (31, 34, 38, 41) de sortie de la lumière est disposé sensiblement transversalement à l'axe longitudinal de l'élément (24) d'application et **en ce que** l'élément (24) d'application comporte, dans la zone de la sortie de la lumière des moyens (32, 39, 40, 41) optiques, qui, avant sa sortie, dévient le faisceau lumineux vers le point (31, 34, 38, 41) de sortie de la lumière, **en ce que** la pointe de travail de l'élément d'application a, dans la région du point de sortie de la lumière, des éléments (35) latéraux de guidage qui dirigent automatiquement le point de sortie de la lumière sur la surface à traiter, l'élément (24) d'application étant à cet effet monté tournant sur une pièce (20) de maintien recevant l'extrémité du guide (11) de lumière.

2. Pièce à main suivant la revendication 1, **caractérisée en ce que** les moyens (32, 39, 40, 41) optiques concentrent la lumière laser dans l'élément (24) d'application de manière que le foyer se trouve juste devant la fenêtre (31, 34, 38) de sortie de la lumière de la pointe de travail.

3. Pièce à main suivant la revendication 1 ou 2, **caractérisée en ce que** la pièce (20) de maintien est fixée de manière à pouvoir être retirée dans le boîtier (18) de tête.

4. Pièce à main suivant la revendication 1 ou 2, **caractérisée en ce que** la pièce (20) de maintien comporte des éléments (23) optiques pour concentrer la lumière sur les éléments (28) de guidage de la lumière dans l'élément (24) d'application.

5. Pièce à main suivant l'une des revendications 1 à 4, **caractérisée en ce que** l'élément d'application est réalisé en conducteur creux ou contient un conducteur de ce genre.

6. Pièce à main suivant l'une des revendications 1 à 5, **caractérisée en ce que** l'élément d'application comporte plusieurs fenêtres (38, 41) de sortie de la lumière ménagées sur le pourtour, ce qui fait que le faisceau de lumière laser peut sortir sur tout le pourtour de la pointe de travail.

7. Pièce à main suivant l'une des revendications 1 à 6, **caractérisée en ce que** l'élément d'application est constitué d'un matériau plein laissant passer de la lumière laser (figures 9 à 11).

8. Pièce à main suivant l'une des revendications 1 à 6, **caractérisée en ce que** les moyens optiques comprennent des surfaces (39, 40) intérieures, qui réfléchissent la lumère laser, qui sont ménagées dans la zone de la pointe de travail et qui fixent le foyer juste avant la fenêtre (38, 41) de sortie de la lumière de la pointe de travail.

9. Pièce à main suivant l'une des revendications 1 à 8, **caractérisée en ce que** la pièce à main est enfilée sur l'armature de raccordement d'un tuyau souple d'alimentation de manière à pouvoir tourner et à pouvoir être retirée.

10. Pièce à main suivant la revendication 9, **caractérisée en ce que** l'armature (1) de raccordement est réalisée en raccord rapide et comporte les moyens de transmission de fluides nécessaires pour la lumière laser, de l'air et éventuellement de l'eau, ainsi que pour du courant électrique servant à éclairer la zone de travail.

11. Pièce à main suivant la revendication 10, **caractérisée en ce que** la transmission de la lumière laser à l'intérieur de la pièce à main par faisceau libre et par déviation sur des miroirs optiques s'effectue au moyens de conducteurs lumineux en matériau plein ou constitués de conduites creuses, au moyen de guides de liquides ou d'une combinaison desdits moyens de transmission.
